# EUROPÄISCHE PATENTSCHRIFT

(11) **EP 0 835 861 B1**
(45) Veröffentlichungstag und Bekanntmachung des Hinweises auf die Patenterteilung: **26.04.2000**
(21) Anmeldenummer: 97114447.2
(22) Anmeldetag: 21.08.1997
(51) Int. Cl.: C07C 67/39, C07C 69/82, C07C 69/76

(54) **Verbesserte Oxidation im Witten-Hercules-Verfahren zur Herstellung von Dimethylterephthalat**
Oxidation in the Witten-Hercules process for the preparation of dimethyl terephthalate
Oxydation dans le procédé Witten-Hercules pour la préparation du téréphtalate de diméthyle

(30) Priorität: 11.10.1996 DE 19641912
(43) Veröffentlichungstag der Anmeldung: 15.04.1998
(73) Patentinhaber: Degussa-Hüls Aktiengesellschaft, 60287 Frankfurt am Main (DE)
(72) Erfinder: Jelko, Stefan, 45721 Haltern (DE); Schoengen, Anton, 58452 Witten (DE); Korte, Hermann-Josef, Dr., 45721 Haltern (DE); Franz, Gerhard, Dr., 45770 Marl (DE); Srebny, Hans-Günther, Dr., 31582 Nienburg (DE); Jostmann, Thomas, Dr., 48249 Dülmen (DE); Steding, Frank, Dr., 45768 Marl (DE)

(56) Entgegenhaltungen:
- EP-A- 0 279 288
- BE-A- 558 857
- DE-A- 3 904 586

## Beschreibung

Die Erfindung betrifft eine verbesserte Verfahrensweise bei der Oxidation im Witten-Hercules-Verfahren zur Herstellung von Dimethylterephthalat (DMT).

### 1. Stand der Technik

Beim Witten-Hercules-Verfahren zur Herstellung von DMT oxidiert man p-Xylol (pX) in einer oder mehreren Stufen in Gegenwart von Kobalt- und Mangankatalysatoren mit sauerstoffhaltigen Gasen, wie Luft, zu p-Toluylsäure (pTS), verestert diese mit Methanol zu p-Toluylsäuremethylester (pTE), der in die Oxidation zurückgeführt wird, wo er zu Terephthalsäuremonomethylester (Monomethylterephthalat; MMT) oxidiert wird. Das Oxidationsprodukt pTS besitzt noch eine weitere oxidierbare Methylgruppe und wird deshalb unter sehr ähnlichen Bedingungen zu Terephthalsäure (TPS) oxidiert wie pTE zu MMT. Da jedoch die Schmelztemperatur der pTS höher als die Oxidationstemperatur ist und die aus ihr entstehende TPS sogar unschmelzbar und zudem im Reaktionsmedium schlecht löslich ist, darf diese Reaktion nur in begrenztem Umfang stattfinden, da das Oxidat sonst nicht mehr handhabbar ist. Es ist daher kennzeichnend für alle Ausführungsformen des Witten-Hercules-Verfahrens, daß in einer Konkurrenzreaktion pTE zu MMT oxidiert wird. MMT ist wesentlich besser löslich als TPS, und pTE ist ein gutes Lösungsmittel für pTS und MMT, hat einen niedrigen Schmelzpunkt und erniedrigt schon in verhältnismäßig geringen Mengen die Schmelztemperatur des Oxidats beträchtlich.

Eine vergleichbare Wirkung üben Kreislaufprodukte aus, die aus nachfolgenden Stufen des DMT-Verfahrens stammen und neben in der Regel weit überwiegenden Anteilen an oxidationsneutralen Stoffen, wie Benzoesäuremethylester (BME), DMT, Dimethyl-o-phthalat (DMO) und Dimethyl-iso-phthalat (DMI), untergeordnete Mengen an oxidierbaren Anteilen, wie Terephthalaldehydsäuremethylester (TAE) und Hydroxymethylbenzoesäuremethylester (HMBME), enthalten. Besonders wirksam in diesem Sinne sind BME, DMO und DMI. Wenn man diese Kreislaufprodukte in die Oxidation des pX einführt, kann man die Menge des gleichfalls eingeführten pTE geringer halten. Dies ist erwünscht, weil die Abtrennung von überschüssigem, d. h. nicht oxidiertem pTE in der auf die Oxidation und die Veresterung folgenden destillativen Aufarbeitungsstufe (der sogenannten Rohesterdestillation) einen hohen Energieaufwand erfordert. Man nimmt daher den hohen Anteil der Kreislaufprodukte an oxidationsneutralen Stoffen als kleineres Übel in Kauf, obwohl sie die Raum-Zeit-Ausbeute herabsetzen und eine Erhöhung der Reaktionstemperatur erfordern. Natürlich müssen in Anlagen mit nur geringen Mengen an Kreislaufprodukten, wie sie in der DE-OS 39 04 586 beschrieben sind, entsprechend höhere Mengen pTE in die pX-Oxidation geführt werden.

In der Veresterungsstufe, die auf die Oxidation folgt, werden MMT und TPS sowie pTS mit Methanol verestert, und das Rohestergemisch mit seinen Hauptbestandteilen DMT und pTE wird durch Destillation getrennt. Das rohe DMT kann durch weitere Destillation und/oder Kristallisation gereinigt werden. Aus DMT kann man durch Hydrolyse auch reine, direkt mit Glykolen veresterbare Terephthalsäure (Handelsbezeichnung PTA) gewinnen. Es ist kennzeichnend für das Witten-Hercules-Verfahren, daß pX und pTE im Gemisch oxidiert und daß pTS sowie TPS und MMT gemeinsam verestert werden.

Während die Oxidation von pX zu pTS nur mit geringen Ausbeuteverlusten verbunden ist, stellt die Oxidation von pTE zu MMT bzw. von pTS zu TPS die größte Verlustquelle des Verfahrens dar. Dies gilt zumindest dann, wenn man gemäß einer üblichen Arbeitsweise das gesamte pX und den gesamten pTE sowie gegebenenfalls die erwähnten Kreislaufprodukte dem Anfang der Oxidationszone zuführt und gemeinsam zusammen mit dem Katalysator die gesamte, ein- oder mehrstufige Oxidationszone durchströmen läßt, in der sie unter gegebenenfalls mehrfacher Frischluftzugabe bis zum gewünschten Umsatz oxidiert werden.

Die Oxidation von pX zu pTS verläuft mit erheblich größerer Geschwindigkeit als die Oxidation von pTE zu MMT bzw. von pTS zu TPS. Daher wird zu Beginn der Oxidation bei moderaten Temperaturen, wie 140 bis 145 °C, zunächst der weitaus größte Teil des pX oxidiert. Für die Wahl der Oxidationstemperatur bestehen enge Grenzen. Unterhalb von 135 °C gehen die Umsätze stark zurück, die Reaktion bricht leicht ab, und es bestehen erhebliche Sicherheitsrisiken wegen möglicher Sauerstoffdurchbrüche mit Überschreiten der Explosionsgrenze. Außerdem kann bei derart niedrigen Temperaturen in den Kühlsystemen der Oxidatoren nur Dampf mit niedrigem Druck erzeugt werden, der nur beschränkt verwendbar ist. Temperaturen > 150 °C sind ebenfalls von Nachteil, da bei den üblichen Reaktionsdrücken von 6 bis 8 bar viel pX und pTE mit dem Abgas ausgetragen werden und zurückgewonnen werden müssen. Außerdem wird bei solchen höheren Temperaturen der Sauerstoff so rasch verbraucht, daß die erwünschte Sauerstoffkonzentration im Abgas von mindestens 2 % unterschritten wird. Sauerstoffarmut im Reaktionsgemisch führt zu vermehrter Bildung von Hochsiedern, wie Di- und Terphenylen, während hohe Temperaturen und Sauerstoffüberschuß die Totaloxidation begünstigen. Di- und Terphenyle stellen Verluste dar, da sie nicht in Wertstoffe umgewandelt werden können.

Die Oxidation von pTE und/oder pTS in nennenswertem Umfang gelingt erst dann, wenn das pX weitgehend oxidiert ist und zudem die Temperatur um 10 bis 25 °C gesteigert wird. Die Temperatur muß um weitere etwa 10 °C angehoben werden, wenn, wie weithin üblich, die erwähnten Kreislaufströme zusammen mit pTE, oder gleichzeitig mit pTE, in den Anfangsbereich der Oxidationszone eingeführt werden. Die Verluste durch Totaloxidation und Hochsiederbildung nehmen dann erheblich zu. Solche Temperaturen sind jedoch derzeit praktisch unvermeidbar, wenn befriedigende Umsätze erreicht werden sollen.

In seiner einfachsten Ausführungsform arbeitet die Oxidationsstufe des Witten-Hercules-Verfahrens diskontinuierlich. Man legt pX und pTE zusammen mit Katalysatorlösung vor, erhitzt und leitet solange Luft ein, bis der gewünschte Oxidationsgrad erreicht ist. Bei der einfachsten kontinuierlichen Ausführungsform werden pX, pTE, Luft und Katalysatorlösung einem einzelnen Oxidator zugeführt, und Oxidationsgemisch wird laufend abgezogen. Diese Ausführungsform arbeitet bei einer bestimmten, vorgegebenen Temperatur, trägt also nicht den Erfordernissen Rechnung, die sich aus den erwähnten unterschiedlichen Oxidationseigenschaften der verschiedenen Bestandteile des Oxidationsgemisches ergeben. Eine Variante der einstufigen Oxidation ist in JP-B2 62/14537 beschrieben. Deren Verfahren sieht zwei parallel angeordnete Oxidationskolonnen vor, von denen die eine mit einem molaren Überschuß an pX und die andere mit einem molaren Überschuß an pTE beschickt wird. Die Temperaturen in den beiden parallelen Oxidatoren können gleich oder verschieden sein und liegen vorzugsweise bei 150 bis 190 °C. Diese Anordnung verbessert die Ausbeute an DMT gegenüber der Arbeitsweise mit einem Einzeloxidator. Die Schrift gibt jedoch keine Lehre über den Zusammenhang zwischen der Zusammensetzung des Oxidationsgemisches in den beiden Oxidatoren und der optimalen Temperatur; auch in den Beispielen werden keine Temperaturen angegeben. Außerdem ist der bevorzugte Temperaturbereich so hoch, daß Totaloxidation und Hochsiederbildung in beträchtlichem Maße auftreten, wie auch die unbefriedigenden Ausbeuten in den Tabellen zu den Beispielen zeigen.

Die gebräuchlichste Anordnung der Oxidatoren bei kontinuierlichen Verfahren ist die von zwei oder vorzugsweise drei in Reihe, also hintereinander angeordneten Oxidatoren. Dabei kann man die Temperatur in den einzelnen Oxidatoren der jeweiligen Zusammensetzung des Oxidationsgemisches sowie sonstigen betrieblichen Gegebenheiten gut anpassen. Es ist weithin üblich, das gesamte pX und und den gesamten pTE, gegebenenfalls zusammen mit den gesamten Kreislaufprodukten, durch die gesamte, durch die Oxidatoren in Teilstufen aufgeteilte Oxidationsstufe zu führen. Die vorliegende Erfindung knüpft an diese Ausführungsform mit in Reihe angeordneten Oxidatoren an.

### 2. Zeichnungen

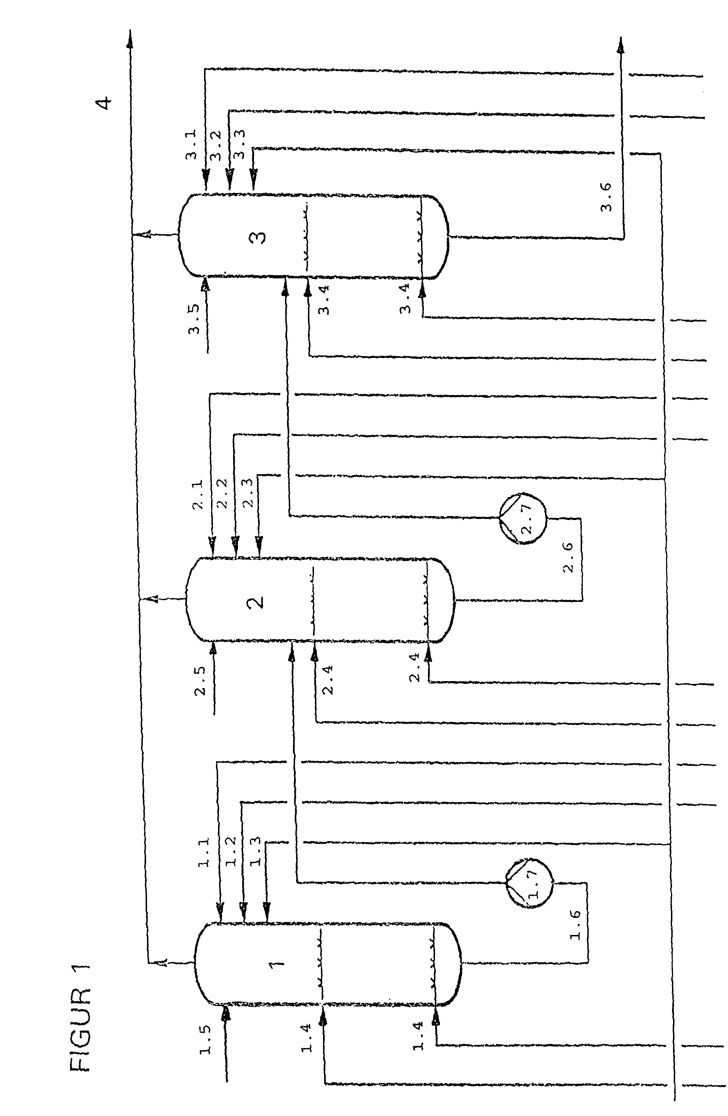

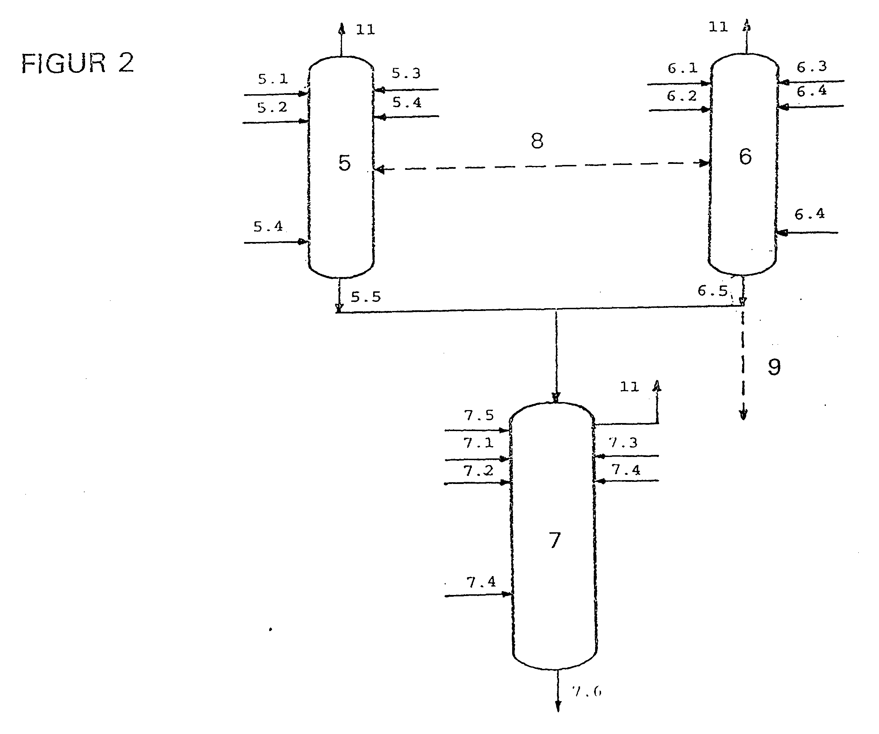

Figur 1 ist ein Schema einer dreistufigen Anlage mit drei in Reihe angeordneten Oxidatoren, in der das Verfahren nach der Erfindung durchgeführt werden kann. Figur 2 ist ein Schema einer zweistufigen Anlage mit drei Oxidatoren, von denen zwei parallel angeordnete Oxidatoren die erste Teilstufe bilden und der dritte Oxidator die zweite Teilstufe darstellt.

### 3. Kurzbeschreibung der Erfindung

Es wurde gefunden, daß sich die Stufe der katalytischen Oxidation von pX und pTE mit sauerstoffhaltigen Gasen im Witten-Hercules-Verfahren zur Herstellung von DMT in mindestens zwei hintereinander angeordneten Teilstufen und in Gegenwart eines Katalysators selektiver gestalten und die Ausbeute an DMT verbessern läßt, wenn man in mindestens einer an pX-reichen Teilstufe einen Teil des pX sowie gegebenenfalls Kreislaufprodukte mit oxidierbaren Anteilen aus Folgestufen des DMT-Verfahrens in Gegenwart von 5 bis 30 Masse-% pTE, bezogen auf das Reaktionsgemisch in der Teilstufe, oxidiert und/oder in mindestens einer weiteren, an pTE-reichen Teilstufe pTE und/oder pTS sowie gegebenenfalls Kreislaufprodukte in Gegenwart von 2 bis 30 Masse-% pX, bezogen auf pTE und pTS, oxidiert.

Die Erfindung gründet sich auf die Beobachtung, daß es vorteilhaft ist, pX in Gegenwart einer begrenzten Menge pTE zu oxidieren und bei der Oxidation von pTE und pTS für die Anwesenheit einer bestimmten Mindestmenge an pX zu sorgen. Es wurde nämlich gefunden, daß pX ein wirkungsvoller H-Donator ist, der dem Zerfall der intermediär entstehenden Peroxy-Radikale und damit der Totaloxidation und der Hochsiederbildung entgegenwirkt. Die Bezeichnungen "an pX-reiche Teilstufe" und "an pTE-reiche Teilstufe" sollen nichts über die absoluten Konzentrationen von pX und pTE in der jeweiligen Stufe aussagen, sondern anzeigen, daß jeweils einer der beiden Stoffe gegenüber dem anderen in überwiegender Menge vorhanden ist. Weiterhin ist die Aussage, daß man in mindestens einer Teilstufe pX und in mindestens einer weiteren Teilstufe pTE oxidiert, nicht so zu verstehen, daß der jeweils andere Stoff in der betreffenden Teilstufe oxidationsneutral wäre. Natürlich werden in beiden Teilstufen beide Stoffe oxidiert, in der an pX-reichen Teilstufe aber vorwiegend pX und in der an pTE-reichen Teilstufe vorwiegend pTE.

### 4. Vorteile der Erfindung

Durch die erfindungsgemäßen Verfahrensmaßnahmen wird eine Reihe von überraschenden Vorteilen erzielt:
(1) Die Selektivität der pX-Oxidation wird um einige Prozentpunkte erhöht. pX spielt seine Rolle als stabilisierender H-Donator offenbar auch dann, wenn in der an pTE-reichen Teilstufe bei den erfindungsgemäßen Konzentrationen und den dann möglichen niedrigeren Temperaturen von < 160 °C oxidiert wird. Weiterhin findet, sofern man die Kreislaufprodukte nicht in die im Verfahrensgang erste Teilstufe einführt, ein Großteil der Oxidation ohne die Kreislaufprodukte statt. Dies ist u. a. deshalb vorteilhaft, weil in den Kreislaufprodukten Stoffe enthalten sind, die die Wirksamkeit des Katalysators herabsetzen. Man kann also niedrigere Temperaturen einstellen, was die Selektivität erhöht, allerdings auch dazu führt, daß in den Kühlsystemen der betreffenden Oxidatoren weniger wertvoller Dampf mit niederem Druck erzeugt wird. Die Möglichkeit, die Reaktionstemperatur innerhalb von Grenzen zu wählen, sichert eine beträchtliche Flexibilität für den Betrieb der Anlage. Je nach den wirtschaftlichen Gegebenheiten - Bedarf an höherwertigem Dampf, Einstandspreis für pX - kann man die optimale Oxidationstemperatur wählen.
(2) Wenn man die Kreislaufprodukte einer Teilstufe, die der im Verfahrensgang ersten, vorzugsweise an pX-reichen Teilstufe nachgeschaltet ist, und insbesondere der letzten Teilstufe zuführt, so daß die Eduktkonzentrationen in der oder den vorhergehenden Teilstufen nicht durch Verdünnung herabgesetzt werden, so kann diese nachgeschaltete Teilstufe mit Temperaturen < 160 °C betrieben werden. Diese Temperaturen reichen überraschenderweise für die Oxidation der leicht oxidierbaren Anteile der Kreislaufprodukte vollkommen aus.
(3) Wegen des hohen Anteils an hochsiedender pTS in der an pX-reichen Teilstufe kann man dort die Temperatur erhöhen, ohne übermäßigen Produktaustrag mit dem Abgas oder ein Aufschäumen des Reaktionsgemisches befürchten zu müssen. Dadurch wird die Raum-Zeit- Ausbeute erhöht und höherwertiger Dampf erzeugt. Die höhere Temperatur geht allerdings auf Kosten der Ausbeute an DMT. Es besteht, wie unter Ziffer (2) erläutert, wiederum die Möglichkeit, die Reaktionstemperatur den jeweiligen technischen und wirtschaftlichen Gegebenheiten anzupassen.
(4) Da die pX-Oxidation in der an pX-reichen Teilstufe wegen der vergleichsweise geringen, aber für die Handhabbarkeit des Oxidats hinreichenden Mengen an zugeführtem pTE weiter fortschreitet als bei vergleichbaren Verfahren nach dem Stand der Technik, werden in dieser Teilstufe und gegebenenfalls in nachfolgenden Teilstufen größere Mengen an TPS erzeugt. Dadurch kann die rückgeführte pTE-Menge vermindert und in der Rohesterdestillation Energie gespart werden.
(5) Die Erfindung läßt sich sowohl bei vorhandenen als auch bei Neuanlagen anwenden, also z. B. bei Anlagen mit zwei oder mehr in Reihe angeordneten konventionellen vertikalen Oxidatoren sowie bei Anlagen mit horizontalen Oxidatoren und Unterteilung in zwei oder mehr Kammern, die den Teilstufen entsprechen. Weiterhin ist es möglich, diskontinuierlich oder parallel betriebene Oxidatoren erfindungsgemäß zu verschalten. Besonders vorteilhaft ist das neue Verfahren bei DMT-Anlagen mit drei in Reihe angeordneten, konventionellen vertikalen Oxidatoren anwendbar. Es ist unerheblich, ob die Anlagen mit Rückstandskreislauf bzw. Thermolyse, mit Methanolyse und/oder mit Isomerenkreislauf betrieben werden. Weiterhin ist unerheblich, ob in den Anlagen DMT und/oder reine Terephthalsäure (PTA) erzeugt wird.

### 5. Beschreibung der Erfindung

### 5.1 Verfahrensparameter

pX ist ein handelsüblicher Rohstoff, der in > 99 % Reinheit verfügbar ist. Je höher der Reinheitsgrad, umso höher der Preis und umso geringer die Menge der entstehenden Nebenprodukte, wie DMO und DMI. Da pX in einem Durchgang zweckmäßig nur zu 50 bis 90 % umgesetzt wird, setzt man zurückgewonnenes pX zusammen mit frischem pX ein. pTE stammt aus der Rohesterdestillation.

Die sauerstoffhaltigen Gase sind in der Regel Luft, die gegebenenfalls mit Sauerstoff angereichert ist. Dadurch wird zwar die Reaktion energischer und die Reaktionsführung erschwert. Die verminderte Abgasmenge trägt aber weniger Wertstoffe aus dem Oxidationsgemisch aus, so daß deren Rückgewinnung weniger aufwendig ist. Die Luftmenge wird vorteilhaft im stöchiometrischen Überschuß angewandt und so bemessen und auf die Teilstufen verteilt, daß das Abgas aus jeder Teilstufe etwa 2 bis 5 Vol.-% Sauerstoff enthält.

Die Oxidationsstufe umfaßt mindestens zwei Teilstufen. Vorteilhaft umfaßt sie drei, gegebenenfalls auch vier und mehr Teilstufen. In mindestens einer Teilstufe muß eine der obigen und/oder-Bedingungen erfüllt sein. In der zweiten Teilstufe kann, aber muß nicht die andere Bedingung erfüllt sein. Es gibt auch Betriebszustände, in denen in einer Teilstufe beide Bedingungen zugleich erfüllt sind. Bei Oxidationsstufen mit drei Teilstufen muß wiederum in mindestens einer Teilstufe mindestens eine der Bedingungen erfüllt sein. Die andere Bedingung kann, aber muß nicht in mindestens einer weiteren Teilstufe erfüllt, sein. Auch hier können die Konzentrationen von pX, pTE und pTS in einer oder auch in mehreren Teilstufen gleichzeitig beiden Bedingungen genügen.

Der Gehalt des Oxidationsgemisches an pTE von 5 bis 30 Masse-% in der mindestens einen an pX-reichen Teilstufe und/oder der Gehalt an pX von 2 bis 30 Masse-%, bezogen auf die Summe von pTE und pTS, in der mindestens einen an pTE-reichen Teilstufe sind wichtige Merkmale des Verfahrens nach der Erfindung. Die Konzentration an pTE in der mindestens einen an pX-reichen Teilstufe beträgt vorteilhaft 5 bis 20 Masse-%, insbesondere 5 bis 15 Masse-%, bezogen auf das Oxidationsgemisch der Teilstufe. Die Konzentration an pX in der mindestens einen weiteren, an pTE-reichen Teilstufe beträgt vorteilhaft ebenfalls 5 bis 20 Masse-% und insbesondere 5 bis 15 Masse-%, aber bezogen auf die Summe von pTE und pTS.

Hinsichtlich des Katalysators entspricht das Verfahren nach der Erfindung dem Stand der Technik. Man verwendet also u. a. wäßrige, gegebenenfalls Carbonsäuren enthaltende Lösungen mit 0,1 bis 10 Masse-% Kobaltsalzen und Mangansalzen, die zweckmäßig aus den im weiteren Verlauf des DMT-Verfahrens anfallenden Destillationsrückständen extrahiert und zum Ausgleich der Verluste mit frischer Lösung ergänzt werden. Man kann auch der oder den im Verfahrensgang letzten Teilstufe(n) katalysatorhaltige Rückstandskreislaufströme zufügen und extrahierte und/oder frische wäßrige Lösung nur in die vorhergehende(n) Teilstufe(n) einführen.

Im Prinzip ist es möglich, Kreislaufprodukte mit den erwähnten oxidierbaren Anteilen z. T. in eine an pX-reiche und auch in eine an pTE-reiche Teilstufe einzuführen. Zweckmäßig führt man jedoch die Kreislaufprodukte in mindestens eine Teilstufe ein, die nach der im Verfahrensgang ersten Teilstufe angeordnet ist. Vorzugsweise führt man die Kreislaufprodukte in die im Verfahrensgang letzte Teilstufe ein. Die Kreislaufprodukte können auch im Gemisch mit pTE in eine passende Teilstufe eingeführt werden.

Der Teil des pX, der in der mindestens einen an pX-reichen Teilstufe oxidiert wird, kann in weiten Grenzen schwanken. Im allgemeinen liegt er bei 25 bis 75 %, bezogen auf umgesetztes pX. Es ist jedoch zweckmäßig, daß in der an pTE-reichen Teilstufe pX in der angegebenen und damit für die Entfaltung der vorteilhaften H-Donatorwirkung ausreichenden Konzentration vorhanden ist.

Die Temperatur in der mindestens einen an pX-reichen Teilstufe 25 liegt im allgemeinen bei 135 bis 155 °C, vorteilhaft bei 140 bis 150 °C. In der mindestens einen weiteren, an pTE-reichen Teilstufe beträgt die Temperatur in der Regel 150 bis 175 °C, vorteilhaft 155 bis 160 °C. Die Oxidation findet in allen Teilstufen im allgemeinen bei Drücken von 2 bis 25 bar, vorteilhaft von 6 bis 8 bar, statt.

### 5.2 Anlagen zur Durchführung des Verfahrens

### 5.2.1 Anlagen mit drei Teilstufen

Im folgenden wird die Anwendung der Erfindung auf die Oxidationsstufe einer DMT-Anlage mit drei in Reihe angeordneten konventionellen vertikalen Oxidatoren beschrieben, die den drei erwähnten Teilstufen entsprechen. Diese Oxidationsstufe ist in Figur 1 schematisch dargestellt. Dabei haben die Oxidatoren **1** bis **3** gleiche Größe. Sie können aber auch unterschiedlich groß sein, was zu unterschiedlichen mittleren Verweilzeiten führt.

Der Oxidator **1**, der der Teilstufe 1 entspricht, besitzt Einleitungen für pX **1.1**, pTE **1.2**, Katalysatorlösung **1.3**, Luft **1.4** als sauerstoffhaltiges Gas und etwaige Kreislaufprodukte **1.5**. Die Luft **1.4** wird hier wie auch bei den folgenden Oxidatoren zweckmäßig auf Einleitungen im unteren Teil des Oxidators sowie im oberen Teil unterhalb des Standes des flüssigen Oxidationsgemisches verteilt.

Die Oxidation von pX und pTE ist stark exotherm. Die Temperatur im Oxidator **1** wird ebenso wie in den folgenden Oxidatoren mittels (in der Figur nicht dargestellter) Wärmetauscher auf der gewünschten Höhe gehalten. Diese Temperatur ist so niedrig, daß bei dem herrschenden Druck das pX **1.1** nicht siedet. Der erzeugte Dampf hat, je nach der Temperatur, einen Druck von 1 bis 8 bar.

Das Oxidationsgemisch **1.6** wird durch die Pumpe **1.7** in den Oxidator **2** geführt, der wiederum mit Einleitungen für pX **2.1**, pTE **2.2**, Kata- lysatorlösung **2.3** und Luft **2.4** versehen ist. Außerdem ist eine Einleitung für Kreislaufprodukte **2.5** mit oxidierbaren Wertstoffen vorhanden.

Das Oxidationsgemisch **2.6** wird aus dem Oxidator **2** durch die Pumpe **2.7** in den Oxidator **3** gefördert, der, wie der Oxidator **2**, mit Einleitungen für pX **3.1**, pTE **3.2**, Katalysatorlösung **3.3**, Luft **3.4** und Kreislaufprodukte **3.5** versehen ist. Das Oxidationsgemisch **3.6** tritt am Boden des Oxidators **3** aus und kann in üblicher Weise weiterverarbeitet werden. Aus den Köpfen der Oxidatoren wird sauerstoffarmes Abgas **4** abgezogen und nach sachgerechter Reinigung unter Rückgewinnung von Wertstoffen in die Atmosphäre entlassen.

In einer solchen dreistufigen Anlage kann z. B. im Oxidator **1** eine an pX-reiche Teilstufe vorliegen. Dann wird die Menge an pTE **1.2** entsprechend bemessen, d. h. gegenüber üblichen Verfahrensweisen vermindert. Dies führt für sich allein zu einer Arbeitsweise nach dem Verfahren der Erfindung, unabhängig davon, wie die Konzentrationsverhältnisse in den nachfolgenden Oxidatoren **2** und **3** sind. Dafür wird in den Oxidator **2** pTE **2.2** in einer Menge eingeführt, die der verminderten Menge an pTE **1.2** entspricht. Man kann auch die fehlende pTE-Menge auf die pTE-Einleitungen **2.2** und **3.2** verteilen, doch wird dies im allgemeinen weniger bevorzugt. In den Oxidator **2** können weiterhin Kreislaufprodukte **2.5** eingeführt werden. Dabei kann es sich um die gesamten anfallenden Kreislaufprodukte handeln, oder diese werden auf die Einleitungen **2.5** und **3.5** verteilt. Es wird jedoch bevorzugt, die gesamten Kreislaufprodukte über die Einleitung **3.5** dem Oxidator **3** zuzuführen. In die Oxidatoren **2** und/oder **3** kann gewünschtenfalls pX **2.1** eingeführt werden, um die erfindungsgemäße Konzentration an pX für mindestens eine weitere, an pTE-reiche Teilstufe in den Oxidatoren **2** und/oder **3** einzustellen. Je nach den Verfahrensparametern in den einzelnen Oxidatoren und insbesondere im Oxidator **1** kann aber diese Konzentration auch ohne weitere Zuführung von pX vorliegen.

Alternativ zu der beschriebenen dreistufigen Variante, in der die im Verfahrensgang erste Teilstufe eine an pX-reiche Teilstufe ist, kann diese erste Teilstufe auch eine an pTE-reiche Teilstufe sein. Dann wird z. B. pTE über die Einleitung **1.2** und pX **1.1** nur in einer solchen Menge zugeführt, daß die erfindungsgemäße Konzentration für die an pTE-reiche Teilstufe herrscht. Die restlichen, überwiegenden Mengen an pX und pTE werden auf die Einleitungen **2.1** bzw. **2.2** und bzw. oder **3.1** bzw. **3.2** verteilt, vorzugsweise aber ausschließlich über die Einleitungen **3.1** bzw. **3.2** zugeführt. Hinsichtlich der Kreislaufprodukte gelten die Ausführungen zu der obigen Verfahrensvariante, bei der die im Verfahrensgang erste Teilstufe eine an pX-reiche Teilstufe ist.

### 5.2.1 Anlagen mit zwei Teilstufen

Für solche Anlagen gelten mutatis mutandis die Erläuterungen zu den dreistufigen Ausführungsformen. Mit zwei Oxidatoren ist die Anlage zwar einfacher und billiger, aber auch weniger flexibel, wenn es um die Optimierung der Verfahrensbedingungen geht. Bei einer Variante des Verfahrens mit zwei Teilstufen umfaßt die erste Teilstufe zwei parallel angeordnete Teilzonen, wobei, wie bei dem Verfahren der erwähnten JP 62/14537, in einer dieser Teilzonen ein molarer Überschuß an pX über pTE und in der anderen Teil-zone ein molarer Überschuß an pTE über pX herrschen kann. Eine entsprechende Anlage mit zwei parallel angeordneten Oxidatoren für die erste Teilstufe und einem dritten, in Reihe angeordneten Oxidator für die zweite Teilstufe ist in Figur 2 schematisch dargestellt. Der Oxidator **5** besitzt Zuführungen für pX **5.1**, pTE **5.2**, Katalysatorlösung **5.3** und Luft **5.4** sowie eine Ableitung für Oxidationsgemisch **5.5.** Entsprechende Zuführungen **6.1**, **6.2, 6.3** und **6.4** sowie eine entsprechende Ableitung **6.5** hat auch der parallel angeordnete Oxidator **6**. Die Oxidatoren **5** und **6** sind durch die Leitung **8** verbunden. durch die Oxidationsgemisch zwischen diesen Oxidatoren ausgetauscht werden kann. Die Oxidationsgemische **5.5** und **6.5** werden in den Oxidator **7** geleitet, der mit den Zuleitungen **7.1**, **7.2, 7.3** und **7.4** versehen ist, entsprechend den Oxidatoren **5** und **6**. Weiterhin werden dem Oxidator **7** Kreislaufprodukte **7.5** zugeführt, und Oxidationsgemisch **7.6** wird entnommen, das zur Veresterung geht. Über die Leitung **9** kann Oxidationsgemisch **6.5** ganz oder teilweise am Oxidator **7** vorbei direkt zur Veresterung geführt werden. Über die Ableitungen **11** wird den Oxidatoren Abgas entnommen, das wie zuvor beschrieben behandelt wird.

Im Oxidator **5** herrscht ein molarer Überschuß von pX über pTE, im Oxidator **6** ist es umgekehrt. Je nach dem Molverhältnis kann in keinem, in einem oder können in beiden Oxidatoren **5** und **6** die erfindungsgemäßen Bedingungen erfüllt sein. Der letztere Fall ist ein Spezialfall der zuvor erwähnten Fall-gestaltung, in der in ein und derselben Teilstufe beide erfindungsgemäße und/oder-Bedingungen erfüllt sind. Wenn in keinem der Oxidatoren **5** und **6** die erfindungsgemäßen Bedingungen herrschen, muß sichergestellt werden, daß die Gegebenheiten im Oxidator **7** erfindungsgemäß sind, z. B. durch Zufuhr von pX **7.1** und/oder Veränderung des Verhältnisses, in dem das Oxidationsgemisch **6.5** auf den Oxidator **7** und die Veresterung aufgeteilt wird. Natürlich kann man durch geeignete Maßnahmen die erfindungsgemäßen Konzentrationen für die an pTE-reiche Teilstufe im dritten Oxidator auch dann einstellen, wenn die Konzentrationen von pX und pTE zumindest in einem der Reaktoren **5** und **6** bereits den erfindungsgemäßen Bedingungen für eine pX-reiche Teilstufe entsprechen.

Wenn die Erfindung auch an den Spezialfällen der Oxidation mit drei in Reihe angeordneten konventionellen vertikalen Oxidatoren und der zweistufigen Oxidation mit parallel angeordneten Reaktoren in der ersten Teilstufe erläutert wurde, so wird der Fachmann dennoch erkennen, daß die erfindungsgemäßen Maßnahmen auch auf andere Oxidationen mit anderer Zahl und/oder Anordnung von Oxidatoren und bzw. oder auf andere Arten von Oxidatoren übertragen werden kann.

### 6. Beispiele

In den folgenden beiden Beispielen werden die bisherige (6.1) und die erfindungsgemäße Betriebsweise (6.2) einer Anlage zur Oxidation mit drei Teilstufen verglichen, wie sie in Figur 1 dargestellt ist. Dabei wurde als Basis für die in der Tabelle wiedergegebenen Massenbilanzen die gleiche Produktionsmenge an DMT gewählt. Die Katalysatorströme sind in beiden Fällen praktisch gleich und wurden in den Massenbilanzen nicht berücksichtigt, da ihre Mengen sehr gering sind.

### 6.1 Bisherige Betriebsweise

Hierbei werden mit Ausnahme von Luft alle Edukte (pX, pTE, Kreislaufströme mit oder ohne oxidierbaren Wertstoffen) in den Oxidator **1** gegeben. Der pX-Strom enthält nicht nur frisches pX, sondern auch pX-reiche Kreislaufströme, die als Kondensate des Oxidationsabgases und der Stripperbrüden anfallen. Diese Kreislaufströme enthalten u. a. pTE in solchen Mengen, daß dessen Anteil im pX-Strom 12 bis 20 Masse-% beträgt. Die weiteren Kreislaufströme 1.5 mit oder ohne oxidierbare Wertstoffen stammen aus verschiedenen nachgeschalteten Anlageteilen, wie Rohesterdestillation und Methanolyse, und enthalten vorwiegend pTE sowie u. a. DMT, BME, HMBME, Methoxymethylbenzoesäuremethylester (MMBME), DMO, DMI und Hochsieder. Das gesamte pTE und alle pTE-haltigen Ströme werden also in den Oxidator **1** eingeführt. Infolgedessen beträgt die Konzentration an pTE im Oxidationsgemisch des Oxidators **1** 35 Masse-%: Bei dieser Betriebsweise beträgt die Ausbeute an DMT, bezogen auf eingesetztes pX, etwa 88 % d. Th.

### 6.2 Betriebsweise nach der Erfindung

Hierbei werden die Edukte nicht vollständig in den Oxidator **1** gegeben, sondern auf die drei Oxidatoren gemäß Tabelle verteilt. Das gesamte pX wird in den Oxidator **1** gegeben. Es enthält, wie erwähnt, pX-reiche und auch pTE enthaltende Kreislaufströme, so daß der pTE-Gehalt im Oxidator **1** 15 Masse-% beträgt. Die überwiegende Menge pTE wird in den Oxidator **2** eingeführt, die Restmenge sowie die Gesamtmenge an Kreislaufströmen mit oxidierbaren und nicht oxidierbaren Wertstoffen in den Oxidator **3**. Bei dieser Betriebsweise beträgt die Ausbeute an DMT, bezogen auf eingesetztes pX, etwa 91 % d. Th.

**Tabelle**

| | Beispiel 6.1 | Beispiel 6.2 |
|---|---|---|
| Strom-Nr. nach Figur 1 | Massenstrom kg/h | Massenstrom kg/h |
| 1.1 | 35.000 | 34.000 |
| 1.2 | 22.400 | - |
| 1.4 | 28.066 | 32.840 |
| 1.5 | 12.600 | - |
| 1.6 | 68.032 | 32.545 |
| 2.2 | - | 20.400 |
| 2.4 | 29.516 | 28.644 |
| 2.6 | 67.518 | 52.996 |
| 3.2 | - | 2.000 |
| 3.4 | 18.961 | 14.399 |
| 3.5 | - | 12.600 |
| 3.6 | 68.599 | 67.918 |
| 4 | 77.944 | 96.905 |

## Patentansprüche

1. Verfahren zur Oxidation von p-Xylol und p-Toluylsäuremethylester mit sauerstoffhaltigen Gasen im Witten-Hercules-Verfahren zur Herstellung von Dimethylterephthalat in mindestens zwei hintereinander angeordneten Teilstufen und in Gegenwart eines Katalysators,
dadurch gekennzeichnet,
daß man in mindestens einer an p-Xylol reichen Teilstufe einen Teil des p-Xylols sowie gegebenenfalls Kreislaufprodukte mit oxidierbaren Anteilen aus Folgestufen des DMT-Verfahrens in Gegenwart von 5 bis 30 Masseprozent p-Toluylsäuremethylester, bezogen auf das Oxidationsgemisch der Teilstufe, oxidiert und/oder in mindestens einer weiteren, an p-Toluylsäuremethylester reichen Teilstufe p-Toluylsäuremethylester und/oder p-Toluylsäure sowie gegebenenfalls Kreislaufprodukte in Gegenwart von 2 bis 30 Masseprozent p-Xylol, bezogen auf die Summe von p-Toluylsäuremethylester und p-Toluylsäure, oxidiert.

2. Verfahren nach Anspruch 1,
dadurch gekennzeichnet,
daß der p-Toluylsäuremethylester in der an p-Xylol reichen Teilstufe in einer Menge von 5 bis 15 Masseprozent, bezogen auf das Oxidationsgemisch der Teilstufe, vorliegt.

3. Verfahren nach Anspruch 1 oder 2,
dadurch gekennzeichnet,
daß das p-Xylol in der an p-Toluylsäuremethylester reichen Teilstufe in einer Menge von 5 bis 15 Masseprozent, bezogen auf p-Toluylsäuremethylester und p-Toluylsäure, vorliegt.

4. Verfahren nach einem der Ansprüche 1 bis 3,
dadurch gekennzeichnet,
daß die an p-Xylol reiche Teilstufe vor der an p-Toluylsäuremethylester reichen Teilstufe angeordnet ist.

5. Verfahren nach einem der Ansprüche 1 bis 3,
dadurch gekennzeichnet,
daß die an p-Toluylsäuremethylester reiche Teilstufe vor der an p-Xylol reichen Teilstufe angeordnet ist.

6. Verfahren nach einem der Ansprüche 1 bis 3,
dadurch gekennzeichnet,
daß man Kreislaufprodukte in mindestens eine Teilstufe einführt, die nach der im Verfahrensgang ersten Teilstufe angeordnet ist.

7. Verfahren nach Anspruch 6,
dadurch gekennzeichnet,
daß die Kreislaufprodukte in die letzte Teilstufe eingeführt werden.

8. Verfahren nach einem der Ansprüche 1 bis 4, 6 oder 7,
dadurch gekennzeichnet,
daß die Oxidation zwei Teilstufen hat, von denen die erste die an p-Xylol reiche und die zweite die an p-Toluylsäuremethylester reiche Tellstufe ist.

9. Verfahren nach einem der Ansprüche 1 bis 3 oder 5 bis 7,
dadurch gekennzeichnet,
daß die Oxidation zwei Teilstufen hat, von denen die erste die an p-Toluylsäuremethylester reiche und die zweite die an p-Xylol reiche Teilstufe ist.

10. Verfahren nach Anspruch 8,
dadurch gekennzeichnet,
daß die erste Teilstufe zwei parallel angeordnete Teiloxidationszonen umfaßt und in einer dieser Teiloxidationszonen ein molarer Überschuß von p-Xylol über p-Toluylsäuremethylester und in der anderen ein molarer Überschuß an p-Toluylsäuremethylester über p-Xylol vorliegt.

11. Verfahren nach Anspruch 10,
dadurch gekennzeichnet,
daß ein Teil des Oxidationsgemisches der ersten Teilstufe vor der Überführung in die zweite Teilstufe von einer der Teiloxidationszonen zur anderen geleitet wird.

12. Verfahren nach Anspruch 10 oder 11,
dadurch gekennzeichnet,
daß ein Teil Oxidationsgemisch aus der an p-Toluylsäuremethylester reichen Teiloxidationszone direkt in die auf die Oxidation folgende Veresterungsstufe geführt wird.

13. Verfahren nach einem der Ansprüche 1 bis 4, 6 oder 7,
dadurch gekennzeichnet,
daß die Oxidation drei Teilstufen umfaßt, wobei die erste Teilstufe eine an p-Xylol reiche Teilstufe und die dritte Teilstufe eine an p-Toluylsäuremethylester reiche Teilstufe ist.

14. Verfahren nach einem der Ansprüche 1 bis 3 oder 5 bis 7,
dadurch gekennzeichnet,
daß die Oxidation drei Teilstufen umfaßt, wobei die erste Teilstufe eine an p-Toluylsäuremethylester reiche Teilstufe und die dritte Teilstufe eine an p-Xylol reiche Teilstufe ist.

## Claims

1. A process for the oxidation of p-xylene and methyl p-toluate with oxygen-containing gases in the Witten-Hercules process for preparing dimethyl terephthalate in at least two sequentially arranged part-stages and in the presence of a catalyst,
characterized in that,
in at least one p-xylene-rich part-stage, some of the p-xylene, and, if desired, recirculated products having oxidizable contents from subsequent stages of the DMT process, is or are oxidized in the presence of from 5 to 30 per cent by mass of methyl p-toluate, based on the oxidation mixture of the part-stage, and/or, in at least one further methyl p-toluate-rich part-stage, methyl p-toluate and/or p-toluic acid, and, if desired, recirculated products, are oxidized in the presence of from 2 to 30 per cent by mass of p-xylene, based on the sum of methyl p-toluate and p-toluic acid.

2. A process according to claim 1,
characterized in that
the methyl p-toluate is present in the p-xylene-rich part-stage in an amount of from 5 to 15 per cent by mass, based on the oxidation mixture of the part-stage.

3. A process according to of either claims 1 and 2,
characterized in that
the p-xylene is present in the methyl p-toluate-rich part-stage in an amount of from 5 to 15 per cent by mass, based on methyl p-toluate and p-toluic acid.

4. A process according to any one of claims 1 to 3,
characterized in that
the p-xylene-rich part-stage is arranged upstream of the methyl p-toluate-rich part-stage.

5. A process according to any one of claims 1 to 3,
characterized in that
the methyl p-toluate-rich part-stage is arranged upstream of the p-xylene-rich part-stage.

6. A process according to any one of claims 1 to 3,
characterized in that
recirculated products are introduced into at least one part-stage which is arranged downstream of the first part-stage in the process sequence.

7. A process according to claim 6,
characterized in that
the recirculated products are introduced into the last part-stage.

8. A process according to any one of claims 1 to 4, 6 and 7,
characterized in that
the oxidation has two part-stages, of which the first is the p-xylene-rich part-stage and the second is the methyl p-toluate-rich part-stage.

9. A process according to any one of claims 1 to 3 and 5 to 7,
characterized in that
the oxidation has two part-stages, of which the first is the methyl p-toluate-rich part-stage and the second is the p-xylene-rich part-stage.

10. A process according to claim 8,
characterized in that
the first part-stage comprises two part-oxidation zones arranged in parallel and in one of these part-oxidation zones there is a molar excess of p-xylene over methyl p-toluate and in the other there is a molar excess of methyl p-toluate over p-xylene.

11. A process according to claim 10,
characterized in that
some of the oxidation mixture of the first part-stage, prior to the transfer to the second part-stage, is passed from one of the part-oxidation zones to the other.

12. A process according co either claim 10 or 11,
characterized in that
some of oxidation mixture from the methyl p-toluate-rich part-oxidation zone is passed directly into the esterification stage which follows the oxidation.

13. A process according to any one of claims 1 to 4, 6 and 7,
characterized in that
the oxidation comprises three part-stages, the first part-stage being a p-xylene-rich part-stage and the third part-stage being a methyl p-toluate-rich part-stage.

14. A process according to any one of claims 1 to 3 and 5 to 7,
characterized in that
the oxidation comprises three part-stages, the first part-stage being a methyl p-toluate-rich part-stage and the third part-stage being a p-xylene-rich part-stage.

## Revendications

1. Procédé d'oxydation du p-xylène et de l'ester méthylique de l'acide p-toluylique avec des gaz contenant de l'oxygène, dans le procédé Witten-Hercules, pour la production de téréphtalate de diméthyle, en au moins deux étapes partielles disposées l'une derrière l'autre, et en présence d'un catalyseur,
caractérisé en ce qu'
on oxyde dans au moins une étape partielle riche en p-xylène, une partie du p-xylène ainsi qu'éventuellement des produits recyclés, avec des fractions oxydables provenant des étapes secondaires du procédé DMT, en présence de 5 à 30 % en masse d'ester méthylique d'acide p-toluylique, rapporté au mélange d'oxydation de l'étape partielle, et/ou on oxyde, dans au moins une autre étape partielle riche en ester méthylique d'acide p-toluylique, l'ester méthylique d'acide p-toluylique et/ou l'acide p-toluylique ainsi que le cas échéant des produits recyclés en présence de 2 à 30 % en masse de p-xylène, rapporté à la somme de l'ester méthylique d'acide p-toluylique et de l'acide p-toluylique.

2. Procédé selon la revendication 1,
caractérisé en ce que
l'ester méthylique d'acide p-toluylique se présente dans l'étape partielle riche en p-xylène, en une quantité allant de 5 à 15 % en masse, rapporté au mélange d'oxydation de l'étape partielle.

3. Procédé selon la revendication 1 ou 2,
caractérisé en ce que
le p-xylène dans l'étape partielle riche en ester méthylique d'acide p-toluylique se présente en une quantité allant de 5 à 15 % en masse, rapporté à l'ester méthylique d'acide p-toluylique et à l'acide p-toluylique.

4. Procédé selon l'une des revendications 1 à 3,
caractérisé en ce que
l'étape partielle riche en p-xylène est disposée avant l'étape partielle riche en ester méthylique d'acide p-toluylique.

5. Procédé selon l'une des revendications 1 à 3,
caractérisé en ce que
l'étape partielle riche en ester méthylique d'acide p-toluylique est disposée avant l'étape partielle riche en p-xylène.

6. Procédé selon l'une des revendications 1 à 3,
caractérisé en ce qu'
on introduit des produits recyclés dans au moins une étape partielle qui est disposée après la première étape partielle dans le cycle opératoire.

7. Procédé selon la revendication 6,
caractérisé en ce que
les produits recyclés sont introduits dans la dernière étape partielle.

8. Procédé selon l'une des revendications 1 à 4, 6 ou 7,
caractérisé en ce que
l'oxydation a deux étapes partielles, parmi lesquelles la première est celle riche en p-xylène et la deuxième l'étape partielle riche en ester méthylique d'acide p-toluylique.

9. Procédé selon l'une des revendications 1 à 3 ou 5 à 7,
caractérisé en ce que
l'oxydation a deux étapes partielles dont la première est une étape partielle riche en ester méthylique d'acide p-toluylique et la deuxième, l'étape partielle riche en p-xylène.

10. Procédé selon la revendication 8,
caractérisé en ce que
- la première étape partielle comprend deux zones d'oxydation partielles disposées en parallèle, et
- dans une des zones d'oxydation partielles, on a un excès molaire de p-xylène par rapport à l'ester méthylique d'acide p-toluylique et, dans l'autre zone, on a un excès molaire d'ester méthylique d'acide p-toluylique par rapport au p-xylène.

11. Procédé selon la revendication 10,
caractérisé en ce qu'
une partie du mélange d'oxydation de la première étape partielle est conduite avant le transfert dans la deuxième étape partielle, d'une des zones d'oxydation partielle à l'autre.

12. Procédé selon la revendication 10 ou 11,
caractérisé en ce qu'
une partie du mélange d'oxydation provenant de la zone d'oxydation partielle riche en ester méthylique d'acide p-toluylique est amenée directement à l'étape d'estérification qui suit l'oxydation.

13. Procédé selon l'une des revendications 1 à 4, 6 ou 7,
caractérisé en ce que
l'oxydation comprend trois étapes partielles, dans lesquelles la première étape partielle est une étape partielle riche en p-xylène, et la troisième étape partielle est une étape partielle riche en ester méthylique d'acide p-toluylique.

14. Procédé selon l'une des revendications 1 à 3 ou 5 à 7,
caractérisé en ce que
l'oxydation comprend trois étapes partielles dans lesquelles la première étape partielle est une étape partielle riche en ester méthylique d'acide p-toluylique et la troisième étape partielle, une étape partielle riche en p-xylène.
